# EUROPEAN PATENT SPECIFICATION

(11) **EP 0 497 933 B1**
(45) Date of publication and mention of the grant of the patent: **24.03.1999**
(21) Application number: 91913534.3
(22) Date of filing: 25.07.1991
(51) Int. Cl.: A61N 2/04

(54) **ELECTRONIC DEVICE FOR SMOOTHING DYSFUNCTIONS OF THE CENTRAL NERVOUS SYSTEM**
ELEKTRONISCHE VORRICHTUNG ZUR KOMPENSATION VON FUNKTIONSTÖRUNGEN DES ZENTRALEN NERVENSYSTEMS
DISPOSITIF ELECTRONIQUE DESTINE A LISSER LES DYSTONIES DU SYSTEME NERVEUX CENTRAL CO

(30) Priority: 24.08.1990 GR 90010063
(43) Date of publication of application: 12.08.1992
(73) Proprietor: ANNINOS, Photios, Prof., GR-681 00 Alexandroupolis (GR); TSAGAS, Nikolaos, Prof., GR-671 00 Xanthi (GR); KOUTSIKOS, Panagiotis, GR-151 Marousi (GR)
(72) Inventor: ANNINOS, Photios, Prof., GR-681 00 Alexandroupolis (GR); TSAGAS, Nikolaos, Prof., GR-671 00 Xanthi (GR); KOUTSIKOS, Panagiotis, GR-151 Marousi (GR)
(74) Representative: Andrikopoulou, Evangelia
(86) International application number: GR9100011
(87) International publication number: WO9203185

(56) References cited:
- EP-A- 0 084 019
- EP-A- 0 099 734
- WO-A-91/06341
- DE-A- 2 707 574
- US-A- 4 940 453
- BERNHARD KRAMER (editor): "THE ART OF MEASUREMENT: Metrology in fundamental and applied physics", 1988, VCH, Weinheim (DE) New York (US); M. Hoke, SQUID-based measuring techniques - A challenge for the functional diagnostics in medicine, see pages 287-333

## Description

### BACKGROUND OF THE INVENTION

The present invention is an electronic device for smoothing dysfunctions of the central nervous system in combination with the use of a Biomagnetometer SQUID. The device in accordance with the invention is defined in claim 1. In a preferred embodiment the electronic device comprises a generator of alternating voltage and low frequency which can produce a given frequency from 2 to 7 Hz and which supplies a given number of selected coils from one or more groups of similar coils for the production of alternating magnetic fields. The intensity of the magnetic fields is regulated by microprocessors. A plurality of generators of alternating voltage and low frequency may be provided. Each of the generators can produce a frequency from 2 to 7 Hz and can supply simultaneously a definite number of selected coils for the production of alternating magnetic fields, having a regulated intensity and frequency by microprocessors. The magnetic fields which are simultaneously produced from the coils must be parallel to the alternating magnetic fields which are emitted from the epileptic foci of the brain. The power spectra and frequencies of the emitted magnetic fields of the coils are of the order of the magnetic fields which are emitted from the epileptic foci, that is from 0.5 pT to 7.5 pT. The limit of the intensities can be extended. Satisfactory results can be obtained using 64 similar coils, which is the number of the measuring points of the left and right brain hemispheres. The epileptic foci are first localized with the use of the SQUID. The present electronic device is adjusted with the use of the SQUID, which gives all of the characteristics of the epileptic foci or any other brain malfunction. Thus, the first step is to localize the epileptic foci with the use of the SQUID, and then to adjust properly the electronic device of the present invention according to the characteristic properties of the localized epileptic foci.

Prior to the present invention were the following publications by the present inventors P.A. Anninos and N.F. Tsagas: Brain Research Bulletin, Vol. 16, 1986, and International Journal of Neuroscience, Vol. 37, 1987, wherein was disclosed the usage of the Biomagnetometer SQUID as a measuring device. This document did not however, disclose anything relevant with the device for treatment of an epileptic patient by inhibiting seizure activity, which is disclosed in the present invention.

Prior to the present invention, the inventors P.A. Anninos and N.F. Tsagas have also filed WO 91/06 341 falling within the terms of Article 54(3) EPC, which referred to the same subject matter of an Electronic device for Smoothing of the Central Nervous System Dysfunctions in Conjunction with the use of the Biomagnetometer SQUID.

The electronic device presented in WO 91/06 341 included a generator of low alternating voltage and frequency which can produce a frequency from 2 Hz to 7Hz, and a defined number of coils of one or more groups of similar coils, in order to produce alternating magnetic fields of defined magnetic field intensity. The device optionally comprised a plurality of generators of alternating voltage and low frequency, each of which could produce a frequency from 2 to 7 Hz, and which could supply simultaneously a defined number of coils in order to produce alternating magnetic fields. The pulse of the alternating voltage could have a square, triangular, sinusoidal or saw-like from. Analogous pulse forms could have alternating magnetic fields which are produced by the coils, the ends of which are connected with the generator output. The number of coils and the cross-section of the coil turns, as well as the shape and composition of the cores, could be varied.

In accordance with WO 91/06 341 the magnetic fields being produced simultaneously from the coils are parallel to the alternating magnetic fields which are emitted from the brain epileptic foci. In addition, the powers and frequencies of the emitted magnetic fields from the coils are of the same order of magnitude as those which are emitted from the epileptic foci. The device is useful to smooth epileptic convulsions in epileptic patients receiving anticonvulsion medication, yet who continue to have seizures. Before the electronic device is used, first the epileptic foci had to be localized with the use of the SQUID.

This early device disclosed in WO91/06 341 however failed to provide or suggest means in effecting the proposed treatment of an epileptic individual with an optimum accuracy and eliminate possible faults connected with the human factor in application of the apparatus.

This problem is solved in the present invention with the inclusion of microprocessor means in the apparatus of the invention and further by providing means for storing the epileptic focal point skull distribution, intensity and frequency information as determined by the SQUID and for inputting the information into the microprocessor means.

Also, the present invention provides in an alternative embodiment a first microprocessor means and a second microprocessor means adapted to control the first microprocessor means to apply an alternating current of appropriate waveform, amplitude and frequency to the appropriate selected coils, which are nearest to the epileptic foci and therefore to generate the appropriate alternating magnetic fields. Further, the second microprocessor means controls any faults of the device and the appropriate selection of the coils, which must be supplied by the appropriate alternating current.

### SUMMARY OF THE INVENTION

The device according to the present invention solves the problem of smoothing the epileptic foci or any other dysfunctions of the central nervous system without the use of the known invasive methods. It is perfectly safe because the applied alternating magnetic fields are of low frequency (from 2-7 Hz) and low intensity (from 0.5 pT to 7.5 pT). This problem is solved with the use of either of one generator of low alternating voltage and frequency which can produce a given frequency from 2-7 Hz and which supplies a given number of selected coils, to produce alternating magnetic fields of which, the intensity and frequency are regulated by microprocessors, or with the use of multiple generators of low alternating voltage and frequency which can each produce a frequency from 2-7 Hz and can supply simultaneously a given number of selected coils for the production of alternating magnetic fields of which the intensity and frequency are adjusted using microprocessors.

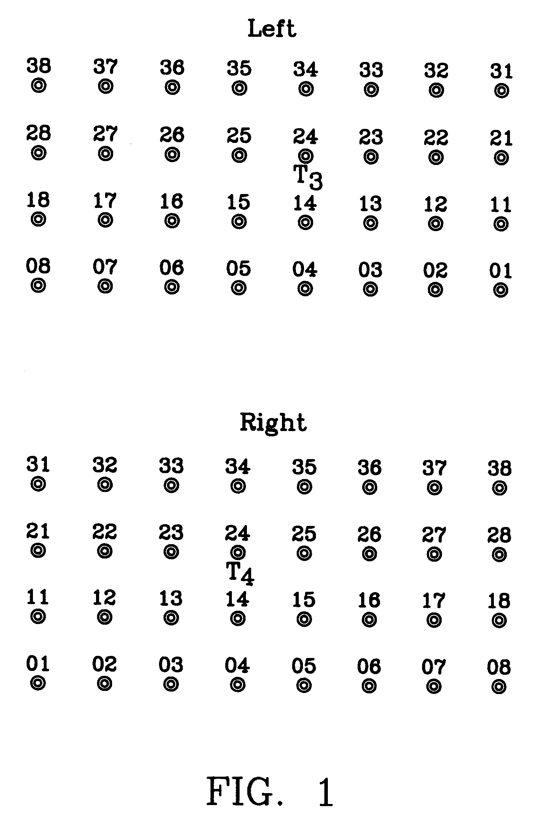

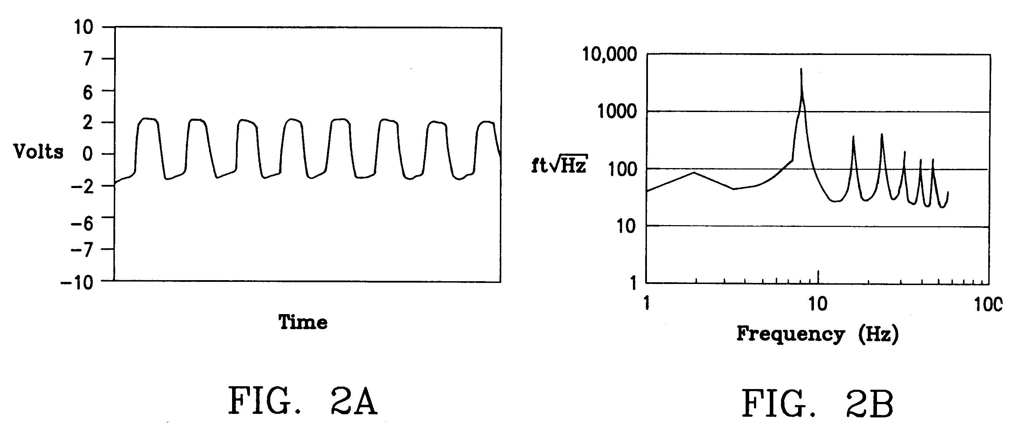

The device is activated with the necessary characteristic elements of the epileptic foci, which are obtained with the use of the biomagnetometer SQUID, and which are properly stored in an integrated circuit of a microprocessor. The proper storage of the data is done using proper software which is written in basic computer language by the present inventors. The computer program reads the data which are stored on the computer disk or diskette during the analysis of the data recorded from the epileptic foci of a patient with the use of the SQUID for all of the 64 or 128 points of skull. These data are stored with the software program in one matrix with three columns, where in the first column are stored the left temporal or occipital or frontal points, and in the other two columns are stored the frequencies and intensities of the magnetic fields which are emitted from each point. The same is done for the right temporal, occipital and frontal regions of the skull. The correspondence between the points is shown in TABLE A and FIG. 1. With the above-described microprocessor placed in the electronic device, its activation results in that every coil emits an alternating square wave magnetic field of a given frequency and intensity as is shown in FIG.2. The figures were obtained by checking every coil using the SQUID, so as to obtain first the wave form of the emitted magnetic field (FIG.2A) and the corresponding power spectrum (FIG. 2B), which gives the fundamental frequency which is emitted from the coil and which must be the same as the frequency and power of the magnetic field which is emitted from the epileptic focus of the corresponding measured point as is stored in the diskette.

The advantage of this method over using a keyboard which would be required in order to enter the data of the epileptic foci and to store them is that the human factor is avoided which otherwise could result in faulty storage of the characteristics of epileptic foci, and furthermore the storage is performed faster depending on the computer running time of the software.

### BRIEF DESCRIPTION OF THE DRAWINGS

In the accompaying drawings:
FIG.1 illustrates the arrangement of measuring points of the left and right temporal hemisphere, respectively, and the reference points T3 and T4, respectively.
FIG.2A shows the waveform of the magnetic field which is emitted from one of the coils of the electronic device of the present invention as was recorded by the biomagnetometer SQUID.
FIG.2B shows the power spectrum of the wave form of FIG.2A.
FIG.3 shows the spiral form and the arrangement of the coils which are used for each hemisphere for smoothing of epileptic foci.
FIG.4 is a schematic diagram of a circuit of the electronic device in accordance with the present invention.
FIG.5 is a block diagram of one of the identical stages used in the present invention.

### DETAILED DESCRIPTION OF THE PREFERRED EMBODIMENT

The present invention is an electronic device, for the smoothing of dysfunctions of the central nervous system in conjunction with a biomagnetometer SQUID. The device comprises one generator of regulated alternating low voltage which produces a given frequency from 2-7 Hz, and which supplies a number of selected coils of one or more groups of similar coils properly arranged to produce alternating magnetic fields. The magnetic fields may be of different wave forms, and the intensity and frequency of the magnetic fields are regulated by microprocessors of multiple generators of regulated alternating low voltage. Each of the multiple generators produces a frequency from 2-7 Hz and the generators supply simultaneously a number of selected coils of one or more groups of similar coils properly arranged in series, which produce alternating magnetic fields of different possible wave forms. The intensity and frequency of the magnetic fields are regulated by microprocessors. The magnetic fields have similar characteristics to those magnetic fields emitted by the epileptic foci, which are determined using the biomagnetometer SQUID.

The effectiveness of the present invention is based on the necessity to use the biomagnetometer SQUID, at least in the first smoothing of the patient during which the first calibration of the electronic device is performed. Points which form one point matrix of rectangular shape (FIG.1) are placed arround the reference points of the 10-20 International Point System for Electrode Placement. The reference points are T3, T4, P3, P4, F3 and F4 for the left or right temporal hemisphere, the left or right occipital regions, and the left or right frontal brain regions, respectively. Thirty-two points are placed on a plastic hat which is placed on the skull of the patient whose reference points have been defined. The thirty-two points are equally spaced by 1.5 cm from each other and are placed at perfectly defined positions on the skull based on the previously defined coordinates of the reference points. By knowing the coordinates of the reference points, then the coordinates of all thirty-two points of the map and, therefore, the coordinates of the epileptic foci are known.

The SQUID sensor is placed 3 mm above each measuring point and thirty-two consecutive records of one second duration are taken from each point and are digitized with a sampling frequency of 256 Hz. Then, Fourier statistical analysis is performed to find the power spectrum of the magnetic amplitude distribution for a given frequency, or a given range of frequencies, using electronic computer techniques. All equal-power spectra amplitudes for a given frequency, or a given frequency domain, are connected to construct ISO-SA maps. From these maps, and from the density of the ISO contour lines, conclusions can be made as to whether epileptic foci are present, as well as their coordinates and spectra power amplitudes. Finally, from this analysis, once the epileptic foci have been localized with the help of spectral analysis, the frequency of the magnetic field emitted from each epileptic foci can be found. These data are stored on a computer diskette, from which with proper software are stored in one microprocessor. Using the microprocessor, it is possible to energize the present electronic device in order to emit back alternating magnetic fields of similar characteristics as those which are emitted from the epileptic foci. Thus, the present device is completely related with the measurements of the SQUID, which is necessary for the calibration of the device. The present electronic device accomplishes in a direct and non-invasive manner the smoothing of the epileptic foci.

The smoothing of epileptic foci using a microprocessor and software eliminates the human factor for the data transfer from the diskette to the microprocessor and, therefore, avoids errors and saves time because the data transfer is accomplished with the computer system. The smoothing and cancellation of epileptic foci remains for several days or months and is based on the influence of the external varying magnetic field, which induces an inhibitory potential in the neuron synapses in the brain regions where the present electronic device is applied.

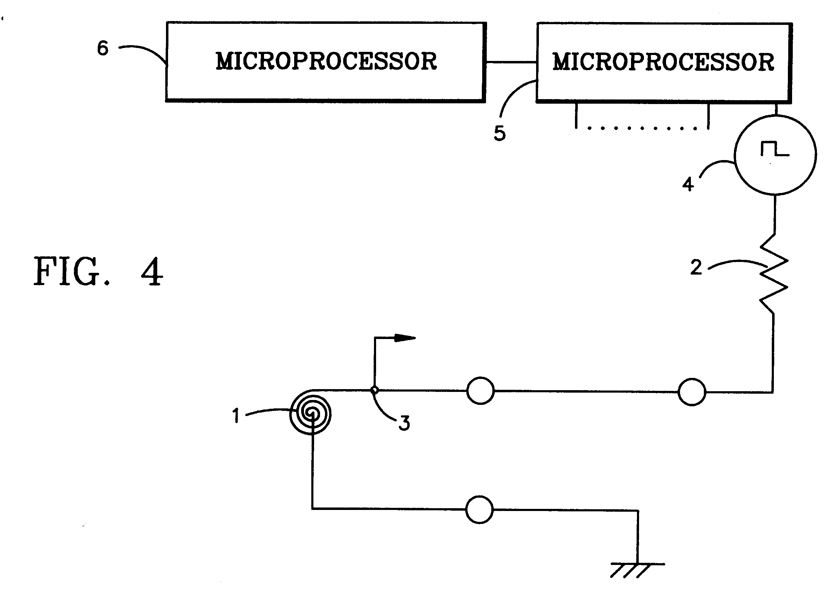

The electronic device comprises m x n circuits, where m is the number of spiral coils made of a flexible metal or alloy of a proper specific resistance which are mounted on n plated made of appropriate flexible material of high strength. The number m can be less than, equal to or more than thirty-two, and the number n can be less than, equal to, or more than four. Referring to FIG.4, each of the circuits is comprised of a spiral coil 1 of which one end is grounded and the other end is connected to an alternating current generator 4 through a resistor 2 and a contact-breaker sensor 3 which activates an alarm system. All the circuits are controlled by a microprocessor 5 which selects and energizes all of the nearest coils to the epileptic foci. These coils are supplied with an alternating current which has all of the appropriate characteristics; namely square or some other wave form, and an amplitude and frequency which have been found to be emitted from the epileptic foci using the biomagnetometer SQUID. All these characteristics are controlled by the microprocessor 5.

The device of the present invention comprises a second integrated circuit of a microprocessor 6 which controls the first microprocessor 5 to apply an alternating current of appropriate waveform, amplitude and frequency to the appropriate selected coils which are nearest to the epileptic foci, and, therefore, to generate the appropriate alternating magnetic fields. Also, the second microprocessor 6 controls any faults of the device and the appropriate selection of the coils which must be supplied by the appropriate alternating current.

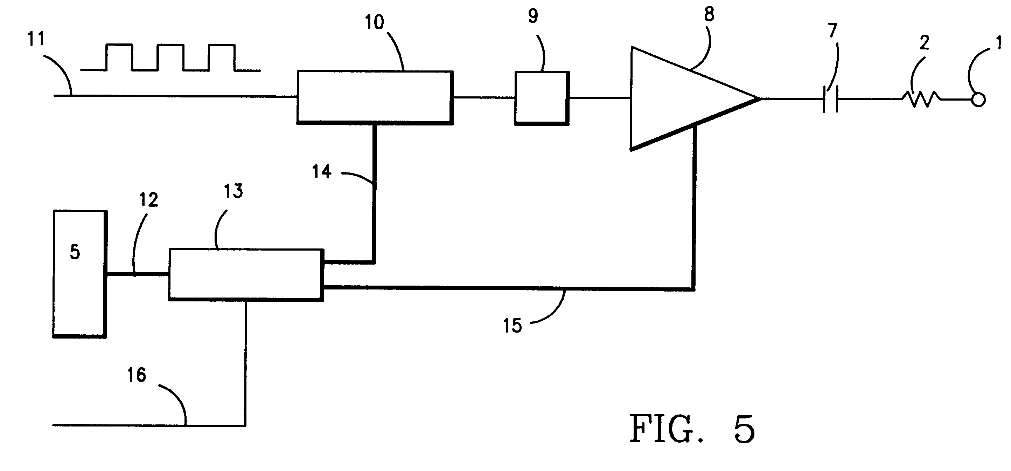

Referring to Fig.5, the individual frequency and amplitude may be set for each pulse train of each spiral coil 1 using a programmable frequency divider 10 and a programmable gain analoque amplifier 8.

FIG.1 illustrates the arrangement of the thirty-two measuring points of the left and right temporal hemisphere, as well as their respective reference points T3 and T4. The same arrangement of the points is used for the measurements of the frontal and occipital hemispheres of an individual.

FIG.2A shows the waveform of the magnetic field which was emitted from one of the sixty-four coils of an electronic device in accordance with the present invention, for the time interval of one second, as was recorded by the SQUID. As shown, the frequency of the emitted magnetic field is 8 Hz.

FIG.2B shows the power spectrum of the wave form of FIG.2A, from which is seen the power amplitude and frequency which was emitted from one of the sixty-four coils, as recorded by the SQUID.

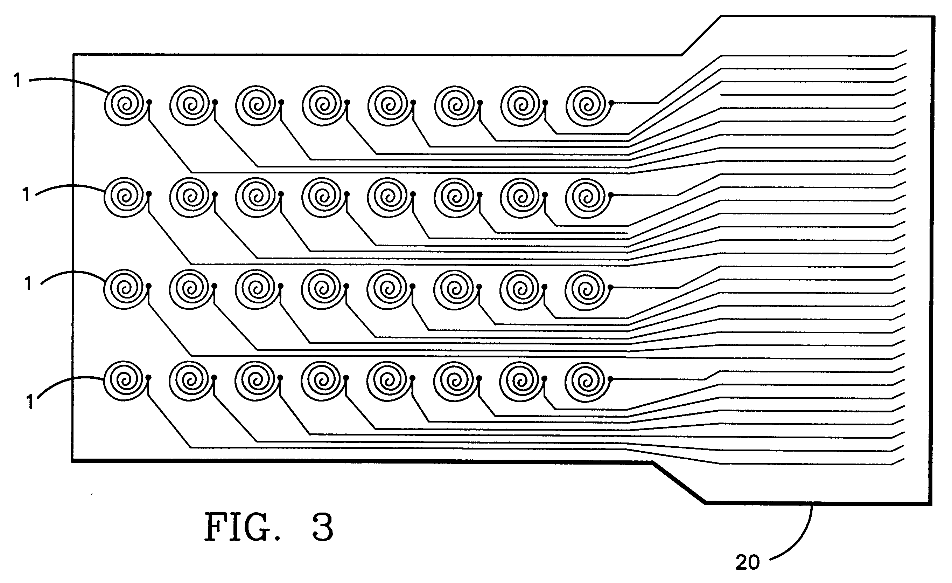

FIG.3 shows the spiral form and the arrangement of the thirty-two coils 1 which are used for each hemisphere for smoothing of epileptic foci. The coils are located on a flexible plate 20.

FIG.4 shows the assembly circuit of the spiral coil 1, which is one of the coils on the flexible plate shown in FIG.3. The resistor 2 of the circuit can have the approximate value of 100 kΩ. The circuit includes a contact-breaker sensor 3 which activates an alarm-system. As explained above, the number of circuits is defined by the number of the coils. The circuit is supplied with an alternating current of square wave or some other wave form 4 which is controlled by the microprocessor 5, This microprocessor controls all of the coils. Also, the microprocessor 5 selects and energizes all the nearest coils to the epileptic foci. These coils are supplied by an alternating current, whose amplitude and frequency are also controlled by the microprocessor 5. The second microprocessor 6 controls the first microprocessor 5 and, in general, controls the normal operation of the device.

FIG.5 is a block diagram of one of the sixty-four identical stages used in the electronic system. Each one of these stages generates a pulse train of specific frequency and amplitude which drives one of the coils. All the stages are connected to a clock input 11 and to a data bus 12 of the microprocessor 5. Each one of the sixty-four stages is also connected to a dedicated enable line 16. As soon as one enable line 16 of the sixty-four stages is driven in low logic level under the control of the microprocessor 5, data from the data bus 12, which is eight bits long, is latched in the corresponding latch 13 until a new enable signal takes place. These eight bits determine the frequency and the amplitude of the pulses of the specific stage. The first four bits are fed through a connection 14 to a programmable frequency divider 10. These four bits determine how much the pulse rate (frequency) is to be reduced. Next, a fixed divider 9 reduces the pulse rate further by a fixed frequency division. Up to this point, the amplitude of the pulses remains unchanged.

The programmable gain amplifier 8 defines the final amplitude of the pulses. The remaining four bits which are latched from the data bus 12 of the microprocessor are used for this purpose. In the same manner, the frequency and the amplitude of all of the sixty-four stages is defined. The number of stages can be less than, equal to, or more than sixty-four.

Table A below gives the recorded characteristics of each of thirty-two points which correspond to epileptic foci and which were stored in one integrated circuit of a microprocessor which energized the electronic device for the magnetic smoothing of epileptic foci. The left and right parts of Table A give the points of the left and right hemisphere of the brain, respectively. The symbols Pt.#, B(PT) and Hz represent the points which were measured on a patient's skull, the amplitudes of the power spectrum in PT, and their frequencies for the smoothing of the epileptic foci, respectively.

**TABLE A**

| **LEFT SIDE** | | | | | | **RIGHT SIDE** | | | | | |
|---|---|---|---|---|---|---|---|---|---|---|---|
| **Pt.#** | **Hz** | **B(T)** | **Pt.#** | **Hz** | **B(T)** | **Pt.#** | **Hz** | **B(T)** | **Pt.#** | **Hz** | **B(T)** |
| 01 | 2 | 6 | 21 | 2 | 6 | 01 | 4 | 6 | 21 | 5 | 6 |
| 02 | 7 | 6 | 22 | 5 | 6 | 02 | 2 | 6 | 22 | 4 | 6 |
| 03 | 3 | 6 | 23 | 5 | 6 | 03 | 5 | 6 | 23 | 4 | 6 |
| 04 | 2 | 6 | 24 | 7 | 6 | 04 | 2 | 6 | 24 | 4 | 6 |
| 05 | 5 | 6 | 25 | 2 | 6 | 05 | 2 | 6 | 25 | 4 | 6 |
| 06 | 5 | 12 | 26 | 7 | 6 | 06 | 2 | 6 | 26 | 7 | 6 |
| 07 | 2 | 12 | 27 | 7 | 12 | 07 | 5 | 6 | 27 | 3 | 12 |
| 08 | 2 | 12 | 28 | 2 | 6 | 08 | 2 | 6 | 28 | 2 | 6 |
| 11 | 2 | 6 | 31 | 2 | 6 | 11 | 2 | 6 | 31 | 5 | 6 |
| 12 | 7 | 12 | 32 | 6 | 6 | 12 | 2 | 6 | 32 | 4 | 6 |
| 13 | 7 | 12 | 33 | 2 | 6 | 13 | 4 | 12 | 33 | 4 | 6 |
| 14 | 2 | 6 | 34 | 2 | 6 | 14 | 2 | 6 | 34 | 4 | 6 |
| 15 | 7 | 6 | 35 | 7 | 6 | 15 | 2 | 6 | 35 | 4 | 6 |
| 16 | 6 | 12 | 36 | 7 | 6 | 16 | 6 | 6 | 36 | 2 | 6 |
| 17 | 2 | 12 | 37 | 2 | 6 | 17 | 2 | 6 | 37 | 2 | 6 |
| 18 | 7 | 6 | 38 | 2 | 6 | 18 | 5 | 6 | 38 | 2 | 6 |

## Claims

1. An electronic apparatus for the treatment of an epileptic individual to inhibit seizure activity, the apparatus comprising:
generating means for generating an alternating voltage and substantially the same frequency as the magnetic field determined to be emitted from each of the individual's epileptic focal points on the skull ;
emitting means electrically connected to said generating means for emitting a magnetic field to the skull of the epileptic individual at the skull coordinates of each epileptic focal point, said emitting means comprising a plurality of coils, and
microprocessor means electrically connected to said emitting means for selecting a number of said plurality of coils to which to apply the generated voltage and for regulating the intensity, frequency and waveform of a current applied to the selected coils such that each selected coil emits a magnetic field having a predetermined distribution, intensity and frequency substantially corresponding to the individual's epileptic focal point skull distribution, intensity and frequency as determined by a superconducting quantum interference device (SQUID).

2. The apparatus of claim 1, wherein said plurality of coils comprises a group of coils having a plurality of rows coils, and each of the rows of coils includes a plurality of coils (1)

3. The apparatus of claim 2, wherein said plurality of coils comprises a plurality of groups of coils (1)

4. The apparatus of claim 3, wherein each of said groups of coils is mounted to a respective flexible plate (20) in a uniformly spaced arrangement.

5. The apparatus of claim 1, wherein each of said plurality of coils (1) is adapted to emit a magnetic field of a frequency of from about 2 Hz to 7 Hz.

6. The apparatus of claim 1, wherein said microprocessor means comprises a first microprocessor means (5) electrically connected to said emitting means for selecting said selected coils (1) and for regulating the intensity, frequency and waveform of a current applied to said selected coils (1)

7. The apparatus of claim 6, wherein said first microprocessor means (5) comprises a programmable frequency divider (10) which regulates the frequency of the current applied to said selected coils (1) and a programmable gain amplifier (8) which regulates the attitude of the applied current.

8. The apparatus of claim 7, wherein said microprocessor means further comprises a second microprocessor means (6) for controlling the perattion of said first microprocessor means (5).

9. The apparatus of claim 1, wherein said generating means comprises one generator (4) adapted to produce and alternating voltage and a frequency of from about 2 Hz to about 7 Hz.

10. The apparatus of claim 1, wherein said generating means comprises a plurality of generators (4) and each generator (4) is adapted to produce and alternating voltage and a frequency of from about 2 Hz to about 7 Hz.

11. The aparatus of claim 1, further comprising means for storing the epileptic focal point skull distribution, intensity and frequency information as determined by the SQUID, and for inputting the stored information into said microprocessor means.

12. The apparatus of claim 1, wherein:
said generating means is a generator means (4) adapted for generating an alternating voltage and a frequency of from about 2 Hz to about 7 Hz;
said emmiting means comprises a plurality of coil means (1) for emitting a magnetic field to the skull of the epiletic individual at the location of the epileptic focal points;
the apparatus further comprising means for storing the epileptic focal point skull distribution, intensity and frequency information as determined by the SQUID, and for inputting the stored information into said microprocessor means.

13. The apparatus of claim 1, wherein:
said generating means comprises a plurarity of generator means (4) each of which is adapted to generate an alternating voltage and a frequency of from about 2 Hz to about 7 Hz;
said emmiting means comprises a plularity of groups of coil means for emitting a magnetic field to the skull of the epileptic individual, each of said groups of coil means including a plurality of coils (1) and each of said plurality of coils (1) being adapted to emit a magnetic field of from about 2 Hz to about 7 Hz;
said microprocessor means comprising a first microprocessor means (5) electrically connected to said emitting means for selecting a number of said plurality of coils (1) to which to apply the generated volatage and for regulating the intensity, frequency and waveform of a current applied to the selected coils (1) such that each selected coil (1) emits a magnetic field having a predetermined distribution, intensity and frequency substantially corresponding to the individual's epileptic focal point skull distribution, intensity and frequency as determined by a superconducting quantum interference device (SQUID);
the apparatus further comprising:
means for storing the epileptic focal point skull distribution, intensity and frequency information as determined by the SQUID, and for inputting the stored information into said first microprocessor means; and
a second microprocessor means (6) for controlling the operation of said first microprocessor means (5).

14. The apparatus of claim 13 wherein said first microprocessor means comprises a programmable frequency divider (10) adapted to regulate the frequency of the current applied to the selected coils, (1), and a programmable gain amplifier (8) adapted to regulate the amplitude of the applied current.

15. The apparatus of claim 13 wherein each of said groups of coils (1) is mounted to a respective flexible plate (20) in a uniformly spaced arrangement.

## Patentansprüche

1. Ein elektronisches Gerät zur prophylaktischen Behandlung einer epileptischen Person um sie vor einem Schlaganfall zu bewahren; das Gerät besteht aus:
einem Generator zur Erzeugung von Wechselspannung und von dem fast selben Frequenzbereich des Magnetfeldes, das muß von jedem epileptischen Brennpunkt auf den Schädel der Person abgegeben werden;
einem Abgeber, der an den besagten Generator elektrisch angeschlossen ist, zum Abgeben eines Magnetfeldes auf den Schädel der epileptischen Person auf den Koordinaten jedes epileptischen Brennpunktes; der besagte Abgeber besteht aus vielen Spulen und
einem Mikroprozessor, der an den besagten Abgeber elektrisch angeschlossen ist, zur Auswahl einiger von besagten Spulen, auf die man die erzeugte Wechselspannung anwenden kann, und zur Regulierung von Intensität, Frequenz und Welleforme eines Stromes, das auf die ausgewählten Spulen angewendet ist; der besagte Mikroprozessor ist so adaptiert, daß jede ausgewählte Spule ein Magnetfeld abgibt; das besagte Magnetfeld hat eine vorherbestimmte Verteilung, Intensität und Frequenz, die fast der Verteilung, Intensität und Frequenz der epileptischen Brennpunkten auf dem Schädel der Person entsprechen, wie es durch ein superleitendes Quanten-Störgerät bestimmt wird.

2. Das Gerät nach Patentanspruch 1, wo die besagten Spulen aus einer Gruppe von Spulen bestehen, die wiederum viele Spulenreihen aufweist; jede Spulenreihe besteht aus vielen Spulen (1).

3. Das Gerät nach Patentanspruch 2, wo die besagten Spulen aus vielen Spulengruppen (1) bestehen.

4. Das Gerät nach Patentanspruch 3, wo jede besagte Spulengruppe auf einer jeweiligen biegsamen Platte (20) in einer gleichförmigen Anordnung montiert ist.

5. Das Gerät nach Patentanspruch 1, wo jede besagte Spule (1) ein Magnetfeld des Frequenzbereich von ungefähr 2 bis 7 Hz abgeben kann.

6. Das Gerät nach Patentanspruch 1, wo der besagte Mikroprozessor aus einem ersten Mikroprozessor (5) besteht; der besagte erste Mikroprozessor, der an den besagten Abgeber elektrisch angeschlossen ist, wird zur Auswahl von besagten ausgewählten Spulen (1) und zur Regulierung von Intensität, Frequenz und Welleforme eines Stromes, das auf den besagten ausgewählten Spulen (1) angewendet ist, verwendet.

7. Das Gerät nach Patentanspruch 6, wo der besagte erste Mikroprozessor (5) aus einem Frequenzverteiler (10), der man programmieren kann, und aus einem Verstärker (8), der man auch programmieren kann, besteht; der besagte Frequenzverteiler (10) reguliert die Frequenz des Stromes, das auf die besagten ausgewählten Spulen (1) angewendet ist; der besagte Verstärker (10) reguliert die Haltung des angewendetes Stromes.

8. Das Gerät nach Patentanspruch 7, wo der besagte Mikroprozessor auch aus einem zweiten Mikroprozessor (6) besteht, welcher das Funktionieren des besagten ersten Mikroprozessor (5) reguliert.

9. Das Gerät nach Patentanspruch 1, wo der besagte Generator aus einem anderen Generator (4) besteht; der besagte Generator (4) ist so adaptiert, um eine Wechselspannung und eine Frequenz von ungefähr 2 bis 7 Hz zu erzeugen.

10. Das Gerät nach Patentanspruch 1, wo der besagte Generator aus viele Generatoren (4) besteht und jeder Generator (4) so adaptiert ist, um eine Wechselspannung und eine Frequenz von ungefähr 2 bis 7 Hz zu erzeugen.

11. Das Gerät nach Patentanspruch 1, besteht auch aus einem Speicher, um Information über die Verteilung, Intensität und Frequenz der epileptischen Brennpunkte auf dem Schädel zu speichern und die gespeicherte Information in den besagten Mikroprozessor einzugeben.

12. Das Gerät nach Patentanspruch 1, wo:
der besagte Generator ein Generator (4) ist, der so adaptiert ist, um eine Wechselspannung und eine Frequenz von ungefähr 2 Hz bis 7 Hz zu erzeugen;
der Abgeber aus vielen Spulengeräten besteht, um ein Magnetfeld auf dem Schädel der epileptischen Person auf die Lage der epileptischen Brennpunkte abzugeben;
das Gerät auch aus einem Speicher besteht, um Information über die Verteilung, Intensität und Frequenz der epileptischen Brennpunkte auf dem Schädel zu speichern und die gespeicherte Information in den besagten Mikroprozessor einzugeben.

13. Das Gerät nach Patentanspruch 1, wo:
der besagte Generator aus vielen Generatoren (4) besteht; jeder Generator so adaptiert ist, um eine Wechselspannung und eine Frequenz von ungefähr 2 Hz bis 7 Hz zu erzeugen;
der besagte Abgeber aus vielen Spulengruppen besteht, um ein Magnetfeld auf dem Schädel der epileptischen Person abzugeben; jede besagte Spulengruppe besteht aus vielen Spulen (1) und jede besagte Spule (1) ist so adaptiert, um ein Magnetfeld von ungefähr 2 bis 7 Hz abzugeben;
der besagte Mikroprozessor aus einem ersten Mikroprozessor (5) besteht; der besagte erste Mikroprozessor, der an den besagten Abgeber elektrisch angeschlossen ist, wird zur Auswahl einiger von den besagten Spulen (1), auf die man die erzeugte Wechselspannung anwenden kann, und zur Regulierung von Intensität, Frequenz und Welleforme eines Stromes, das auf die ausgewählten Spulen angewendet ist, verwendet; der besagte Mikroprozessor ist so adaptiert, daß jede ausgewählte Spule ein Magnetfeld abgibt; das besagte Magnetfeld hat eine vorherbestimmte Verteilung, Intensität und Frequenz, die fast der Verteilung, Intensität und Frequenz der epileptischen Brennpunkten auf dem Schädel der Person entsprechen, wie es durch ein superleitendes Quanten-Störgerät bestimmt wird;
das Gerät besteht auch aus:
einem Speicher, um Information über die Verteilung, Intensität und Frequenz der epileptischen Brennpunkte auf dem Schädel zu speichern, wie es durch das superleitende Quanten-Störgerät bestimmt wird, und die gespeicherte Information in den besagten ersten Mikroprozessor einzugeben; und
einem zweiten Mikroprozessor (6) zur Regulierung des Funktionieren der besagten ersten Mikroprozessor (5).

14. Das Gerät nach Patentanspruch 13, wo der besagte erste Mikroprozessor aus einem Frequenzverteiler (10), der man programmieren kann, und aus einem Gain Verstärker (8), der man auch programmieren kann, besteht; der besagte Frequenzverteiler (10) reguliert die Frequenz des Stromes, das auf die besagten ausgewählten Spulen (1) angewendet ist; der besagte Verstärker (10) ist so adaptiert, um die Haltung des angewendetes Stromes zu regulieren.

15. Das Gerät nach Patentanspruch 13, wo jede besagte Spulengruppe (1) auf einer jeweiligen biegsamen Platte (20) in einer gleichförmigen Anordnung montiert ist.

## Revendications

1. Un appareil électronique pour le traitement d'une personne épileptique afin d'éviter une crise, l'appareil comprenant:
un moyen de génération de tension alternative et essentiellement de la même fréquence du champ magnétique qui doit être émise par tous les points focaux épileptiques de la personne sur le crâne;
un moyen d'émission connecté électriquement audit moyen de génération pour émettre un champ magnétique au crâne de la personne épileptique aux coordonnées de chaque point focal épileptique sur le crâne, ledit moyen d'émission comprenant une pluralité de bobines, et
un microprocesseur connecté électriquement audit moyen d'émission pour sélectionner un nombre de ladite pluralité de bobines sur lequel on doit appliquer la tension générée et pour régler l'intensité, la fréquence et la forme d'onde du courant appliqué aux bobines sélectionnées, tel que chaque bobine sélectionnée émet un champ magnétique ayant une distribution, une intensité et une fréquence prédéterminées correspondant essentiellement à la distribution, l'intensité et la fréquence des points focaux épileptiques sur le crâne de la personne comme il est déterminé par un supraconducteur d'interférence quantique (SQUID).

2. L'appareil de la revendication 1, où ladite pluralité de bobines comprend un groupe de bobines ayant une pluralité de files de bobines, chacune file de bobines comprenant une pluralité de bobines (1)

3. L'appareil de la revendication 2, où ladite pluralité de bobines comprend une pluralité de groupes de bobines (1)

4. L'appareil de la revendication 3, où chacun desdits groupes de bobines est monté sur une plaque flexible respective (20) dans une disposition uniformément espacée.

5. L'appareil de la revendication 1, où chacune de ladite pluralité de bobines (1) est adaptée pour émettre un champ magnétique d'une fréquence de l'ordre de 2 Hz environ à 7 Hz environ.

6. L'appareil de la revendication 1, où ledit microprocesseur comprend un premier microprocesseur (5) connecté électriquement audit moyen d'émission pour sélectionner lesdites bobines sélectionnées (1) et pour régler l'intensité, la fréquence et la forme d'onde d'un courant appliqué sur lesdites bobines sélectionnées (1)

7. L'appareil de la revendication 1, où ledit premier microprocesseur (5) comprend un répartiteur de fréquence programmable (10) réglant la fréquence du courant appliqué aux dites bobines sélectionnées (1) et un amplificateur de gain programmable (8) réglant l'attitude du courant appliqué.

8. L'appareil de la revendication 7, où ledit microprocesseur comprend en outre un deuxième microprocesseur (6) pour contrôler le fonctionnement dudit premier microprocesseur (5).

9. L'appareil de la revendication 1, où ledit moyen de génération comprend une génératrice (4) adaptée à la génération d'une tension alternative et d'une fréquence de l'ordre de 2 Hz environ à 7 Hz environ.

10. L'appareil de la revendication 1, où ledit moyen de génération comprend une pluralité de génératrices (4) et chaque génératrice (4) est adaptée à la génération d'une tension alternative et d'une fréquence de l'ordre de 2 Hz environ à 7 Hz environ.

11. L'appareil de la revendication 1, comprenant en outre un moyen de mise en réserve des données concernant la distribution, l'intensité et la fréquence des points focaux épileptiques sur le crâne comme il est déterminé par le SQUID et d'introduction desdites données mises en réserve dans ledit microprocesseur.

12. L'appareil de la revendication 1, où:
ledit moyen de génération est une génératrice (4) adaptée à la génération d'une tension alternative et d'une fréquence de l'ordre de 2 Hz environ à 7 Hz environ;
ledit moyen d'émission comprend une pluralité de moyens de bobines (1) pour l'émission d'un champ magnétique sur le crâne de la personne épileptique au lieu où se trouvent les points focaux épileptiques;
l'appareil comprenant en outre un moyen de mise en réserve des données concernant la distribution, l'intensité et la fréquence des points focaux épileptiques sur le crâne comme il est déterminé par le SQUID, et d'introduction des données mises en réserve dans ledit microprocesseur.

13. L'appareil de la revendication 1, où:
ledit moyen de génération comprend une pluralité de génératrices (4) chacune desquelles est adaptée à la génération d'une tension alternative et d'une fréquence de l'ordre de 2 Hz environ à 7 Hz environ;
ledit moyen d'émission comprend une pluralité des groupes de moyens de bobines pour l'émission d'un champ magnétique au crâne d'une personne épileptique, chacun desdits groupes de moyens de bobines comprenant une pluralité de bobines (1) et chacune de ladite pluralité de bobines (1) étant adaptée pour l'émission d'un champ magnétique de l'ordre de 2 Hz environ à 7 Hz environ;
ledit microprocesseur comprenant un premier microprocesseur (5) connecté électriquement audit moyen d'émission pour sélectionner un nombre de ladite pluralité de bobines (1) auquel on doit appliquer la tension générée et pour régler l'intensité, la fréquence et la forme d'onde d'un courant appliqué aux bobines sélectionnées (1) tel que chaque bobine sélectionnée (1) émet un champ magnétique ayant une distribution, une intensité et une fréquence prédéterminées et correspondant essentiellement à la distribution, l'intensité et la fréquence des points focaux épileptiques sur le crâne de la personne comme il est déterminé par un supraconducteur d'interférence quantique (SQUID);
l'appareil comprenant en outre:
un moyen de mise en réserve des données concernant la distribution, l'intensité et la fréquence des points focaux épileptiques sur le crâne comme il est déterminé par le SQUID, et d'introduction des données mises en réserve dans ledit premier microprocesseur (5).
un deuxième microprocesseur (6) contrôlant le fonctionnement dudit premier microprocesseur (5).

14. L'appareil de la revendication 13 où ledit premier microprocesseur comprend un répartiteur de fréquence programmable (10) adapté pour régler la fréquence du courant appliqué aux dites bobines sélectionnées (1) et un amplificateur de gain programmable (8) adapté à la régulation de l'attitude du courant appliqué.

15. L'appareil de la revendication 13 où chacun des groupes de bobines (1) est monté sur une plaque flexible respective (20) dans une disposition uniformément espacée.
